# EUROPÄISCHE PATENTANMELDUNG

(11) **EP 0 623 361 A2**
(43) Veröffentlichungstag der Anmeldung: **09.11.1994**
(21) Anmeldenummer: 94105103.9
(22) Anmeldetag: 31.03.1994
(51) Int. Cl.: A61M 39/04, A61M 5/20, A61M 5/31, A61M 5/32

(54) **Portkanülenanordnung zum Anschluss an einen Port**

(30) Priorität: 08.04.1993 DE 4311715
(71) Anmelder: Fresenius AG, D-61350 Bad Homburg (DE)
(72) Erfinder: Wolbring, Peter, Dr., D-66606 St. Wendel (DE); Anderheiden, Dirk, Dr., D-61250 Usingen (DE)
(74) Vertreter: Fuchs, Luderschmidt & Partner Patentanwälte

(57) **Zusammenfassung**

Hauptkanülenanordnung (10) mit einer metallischen Kanüle (12) und einem Anschlußstück (14), das ein Ventil (46) aufweist, das im nicht konnektierten Zustand geschlossen und im konnektierten Zustand geöffnet ist.

## Beschreibung

Die Erfindung betrifft eine Portkanülenanordnung zum Anschluß an einen Port, die eine Portkanüle aus Metall mit einem Anschlußstück an ihrem proximalen Ende, das einen weiblichen Luer-Konus zur Aufnahme eines Schlauchsystems aufweist, und an ihrem distalen Ende angeschliffen ist, sowie eine Ventilanordnung zum Öffnen und Verschließn des Zugangs der Portkanüle aufweist.

Portkanülen werden zur Herstellung eines Zugangs zu einem implantierten Portsystem verwendet. Ein solches Portsystem dient üblicherweise als intrakorporaler Zugang zur Applikation von flüssigen Medikamenten und dergleichen, die über einen Katheterschlauch, der mit dem Port in Verbindung steht, an ein Blutgefäß abgegeben werden. Ein solches Portsystem stellt somit selbst den Zugang zu einer Vene dar und weist durch sein abgeschlossenes Septum bzw. seine Kapillaranordnung Ventilfunktion auf.

Übliche Portkanülensysteme weisen eine Portkanüle aus Metall auf, die an ihrem distalen Ende über ein Anschlußstück verfügt, das wiederum mit einem Innenkonus (üblicherweise ein weiblicher Luer-Konus) versehen ist. Somit steht der lennenkanal dieses Anschlußstücks mit dem Kanülenkanal in Strömungsverbindung, ohne daß irgendwelche Absperrorgane dort vorgesehen sind. An dieses Anschlußstück wird üblicherweise ein Infusionsleitungssystem mittels eines männlichen Luer-Anschlußstücks angeschlossen, über das zu infundierende Medikamente und dergleichen dem Patienten zugeführt werden. Bei einem von der Anmelderin vertriebenen System mit der Bezeichnung "IN-TRAPORT" ist in der Infusionsleitung ein 3-Wege-Hahn vorgesehen, um die Durchführung mehrerer Injektionen bei der Medikamentenapplikation oder der Entnahme einer Blutprobe über das implantierte Kathetersystem möglich zu machen. So wird in der Klinischen Praxis die Punktionskanüle zunächst mit dem Absperrhahn verbunden, dann eine z.B. mit Kochsalzlösung gefüllte Spritze aufgesetzt und das Injektionssystem entlüftet. Mit dem entlüfteten System wird der Port punktiert und gespült, um feststellen zu können, ob das implantierte Kathetersystem funktionsfähig ist. Danach wird der Hahn geschlossen, die Spritze entfernt, eine weitere Spritze aufgesetzt, die dann das zu verabreichende Medikament enthält, der Hahn wieder geöffnet und das Medikament injiziert. Danach wird der Hahn erneut geschlossen, eine meist mit heparinisierter Kochsalzlösung gefüllte Spritze aufgesetzt, der Hahn geöffnet und ein Heparin-Block gesetzt, damit das Kathetersystem nicht durch Thrombenbildung verstopft und unbrauchbar wird. Schließlich wird die Kanüle danach aus dem Portgehäuse herausgezogen, wobei ein leichter Überdruck in der Spritze aufrechterhalten werden muß, so lange sich die Kanülenspitze noch im Portgehäuse befindet. Diese Arbeitsweise ist bei der Aspiration von Blut im wesentlichen identisch.

Die Handhabung dieses Systems bei der Injektion von Medikamenten oder der Aspiration von Blut ist sehr umständlich, so daß eine große Gefahr von Handhabungsfehlern existiert.

Rückflußverhindernde Katheter- oder Kanülenventile sind jedoch seit langer Zeit bekannt und werden beispielsweise bei dem Kathetersystem der Anmelderin mit der Bezeichnung "CAVATHETER" seit Jahren verwendet. Einerseits sind derartige Ventile im Katheteransatz, andererseits im Ansatz der PTFE-Kanüle vorgesehen, durch deren Kapillare der Katheter eingeführt wird. Sie haben in beiden Fällen die Aufgabe, den Blutrückfluß bei der Katheterplazierung zu verhindern, und darüber hinaus der Gefahr von Luftembolien entgegenzuwirken. Derartige Kathetersysteme sind beispielsweise aus den deutschen Patentschriften 28 17 102, 29 18 326 oder 30 00 903 bekannt, in denen jeweils ein Anschlußstück für eine Kanüle beschrieben ist, die jeweils über eine geschlitzte Ventilscheibe verfügen. Die vorstehend genannten Handhabungsprobleme werden jedoch mit diesen Anschlußstücken nicht beseitigt, da diese auf die Portkanüle aufgesetzt werden müssen.

Andererseits sind auch einstückige Kanülen mit Rückschlagventil vorgeschlagen worden, beispielsweise in der DE 41 37 019 C. Hier befindet sich ein Ventil direkt in der Kanüle, was einerseits technisch nur schwierig realisierbar ist und andererseits lediglich bei Venenkanülen anwendbar ist, bei denen eine gewisse Dicke der Venen vorausgesetzt werden kann. Aus der DE 42 04 002 A ist eine Vorrichtung zur Blutentnahme und zur Injektion zu entnehmen, zu der auch eine Portkanüle zu rechnen ist. Die hieraus bekannte Kanüle wird mit Hilfe einer Sprungfeder durch eine Gummimembran gestoßen, die bei einer Portkanüle jedoch nicht eingesetzt werden kann. Bei jedem Durchstechen der Membran werden nämlich Partikel herausgestanzt, die entweder den Portkatheter zusetzen oder aber in den Patienten gespült werden können. Insofern werden spezielle Portkanülen (beispielsweise die sogenannte Huber-Nadel) für den Portdurchstich verwendet, wobei versucht wird, den Port möglichst selten anzustechen, um möglichst wenig Partikel zu erzeugen. Insofern sollte bei verschiedenen Handgriffen die Portkanüle liegen bleiben.

Der Erfindung liegt daher die Aufgabe zugrunde, ein Portkanülensystem der eingangs erwähnten Art zu schaffen, welches für den behandelnden Arzt möglichst wenig Handhabungsprobleme aufweist und das dem Patienten möglichst geringe Schmerzen verursacht, die durch die fortwährende Bewegung der eingeführten Kanüle verursacht werden.

Die Lösung der Aufgabe gelingt durch die kennzeichnenden Merkmale des Anspruchs 1.

Das erfindungsgemäße Kanülensystem besteht aus zwei Funktionselementen, nämlich einerseits der Injektionskanüle selbst, die auch eine Spezialkanüle z.B. für die Punktion implantierter Port- oder Kathetersysteme sein kann, und andererseits aus dem im Anschlußstück der Kanüle vorgesehenen Ventil, das durch den männlichen Luer-Konus einer aufgesetzten Spritze oder eines angeschlossenen Schlauchsystems unter Öffnung des Ventils betätigt wird. Beim Abziehen des konusförmigen Anschlußsystems sperrt das Ventil den Rückfluß von Flüssigkeiten zwangsläufig ab. Insofern wird ein Rückfluß dieser Flüssigkeit nach der Punktion natürlicher oder künstlich angelegter Körperhohlräume verhindert.

Durch die Anordnung des erfindungsgemäßen Rückschlagventils kann auf den beim INTRAPORT-System eingesetzten 3-Wege-Hahn verzichtet werden. Zugleich sperrt dieses Rückschlagventil beim Abziehen einer Spritze oder eines Schlauchsystems unmittelbar die Portkanüle ab, so daß kein weiterer Unterdruck mehr erzeugt wird, der zu einem Rückziehen von Körperflüssigkeit führen würde.

Die Anordnung des erfindungsgemäßen Rückschlagventils in dem Anschlußstück ist insofern überraschend, als bisher die Kombination des bei Katheter bekannten Rückschlagventils mit einer Portkanüle aus Stahl nicht zweckmäßig erschien, da dem Port üblicherweise nur Flüssigkeiten zugeführt werden und der Einsatz eines 3-Wege-Ventils ausreichend erschien.

Es sei insbesondere zweckmäßig, direkt an der Verbindungsstelle der Portkanüle einerseits und des zu verbindenden Systems andererseits diese Sperre anzubringen, da die beiderseits der Ventilsperre befindlichen Kanäle sicher mit Infusionsflüssigkeit gefüllt werden können, so daß sicher die Gefahr von Luftembolien vermieden werden kann. Letzteres ist bei offenen Systemen nicht gesichert, da durch die auftretenden Unter- oder Überdrücke insbesondere die Portkanüle häufig nicht vollständig gefüllt ist, so daß besondere Vorkehrungen zur Entfernung der in der Kanüle verbliebenen Luft getroffen werden müssen.

Wird also das erfindungsgemäße Ventil anstatt des bisher üblichen Hahns bei der Portkanüle verwendet, so entfallen alle Handgriffe, die für die Bedienung des Hahns notwendig sind.

Neben der Injektion von Medikamenten in ein Portsystem oder eine implantierte Pumpe sind weitere Anwendungen möglich, die sich durch die Gemeinsamkeit auszeichnen, daß sich einer Punktion mit Hilfe einer Kanüle mehrere Arbeitsgänge anschließen, die einen Wechsel der extrakorporalen Geräte zwingend erforderlich machen. So ist beispielsweise die perkutane Entnahme von Urin aus der Blase zu diagnostischen Zwecken mit dem erfindungsgemäßen System möglich. Desgleichen können größere Mengen arteriellen Blutes zur Blutgasanalyse mit dem erfindungsgemäßen System entnommen werden.

Vorteilhafte Ausgestaltungen der Erfindung sind Gegenstand der Patentansprüche 2 und 3.

Ein Ausführungsbeispiel der Erfindung wird unter Bezugnahme auf die Zeichnung nachstehend erläutert.

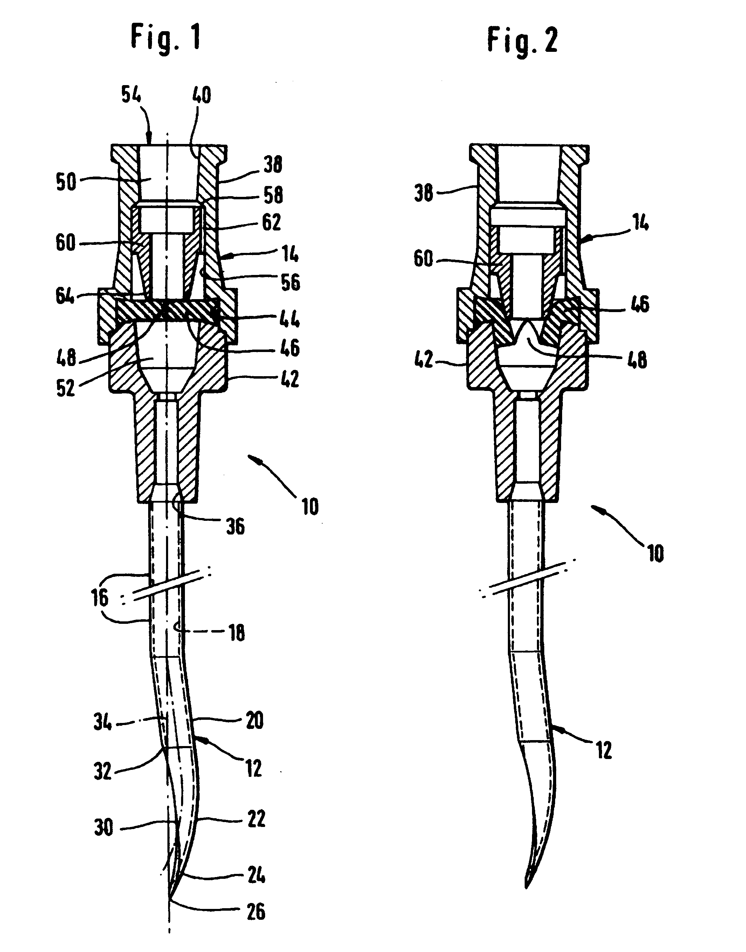

Es zeigt
Figur 1 eine Portkanülenanordnung, wobei die Portkanüle selbst in der Seitenansicht und das Anschlußstück im Querschnitt dargestellt sind, sowie Figur 2 die Kanülenanordnung mit geöffneter Membran.

In Figur 1 und 2 ist die Portkanülenanordnung mit 10 bezeichnet, die eine Portkanüle 12 und ein Anschlußstück 14 aufweist.

Die Portkanüle 12 weist einen im wesentlichen geraden Abschnitt 16 mit einem gestrichelt dargestellten Durchgangskanal 18 auf. Dieser Abschnitt 16 mündet an seinem distalen Ende in einem seitlich abgeknickten Endabschnitt 20, wobei der Durchgangskanal 18 in einem Bogenabschnitt 22 sich fortsetzt und letztlich in ein Einstechteil 24 mit einer Spitze 26 ausläuft. Dabei ist in dem Einstechteil eine Lumenöffnung 30 vorgesehen, die vorteilhafterweise linsenförmig ausgebildet ist, jedoch im Bereich der Spitze 26 in einer Öffnungspitze ausläuft, während das der Öffnungsspitze gegenüberliegende Ende gerundet ist. Die Lumenöffnung 30 ist weiterhin in der konkaven Seite des Bogenabschnitts 22 angeordnet und weist eine dem Endabschnitt 20 benachbarte einwärts in den Bogenabschnitt 22 errichtete Hinterkante 32 auf. Dabei liegt die Spitze 26 und die Hinterkante 32 auf der gedachten Zentralachse 34 des Durchgangskanals 18.

Die Form der Portkanüle 12 ist jedoch nicht kritisch, bevorzugt ist jedoch die Kanüle, die in der DE 41 01 231 A beschrieben ist, auf die aus Offenbarungsgründen Bezug genommen wird.

Das proximale Ende 36 der Portkanüle 12 mündet in dem Anschlußstück 14. Dieses Anschlußstück 14 besteht aus einem ersten Verbindungsteil 38, das einen Luer-Innenkonus 40 aufweist, und einem vorderen zweiten Verbindungsteil 42, das mit dem proximalen Ende 36 der Portkanüle 12 verbunden ist. Beide Verbindungsteile 38 und 42 sind miteinander verbunden und verschweißt, wobei im zusammengefügten Zustand im Verbindungsbereich eine radial umlaufende Nut 44 gebildet wird, in der eine kreisscheibenförmige Ventilscheibe 46 aus elastomerem Material gehaltert ist. Diese Ventilscheibe 46 ist mit einem zentralen Schlitz 48 versehen, der beiderseits vor dem Rand der Scheibe 46 endet.

Sowohl das erste Verbindungsteil 38 als auch das zweite Verbindungsteil 42 weisen jeweils einen zentralen Durchtrittskanal 50 bzw. 52 auf, die bei geöffnetem Schlitz 48 miteinander und mit dem Durchgangskanal 18 der Portkanüle 12 in Strömungsverbindung sind.

Gemäß der in Figur 1 gezeigten Ausführungsform schließt sich an den weiblichen Innenkonus 40 ein zylindrischer Führungsabschnitt 56 an, dessen Durchmesser größer ist als der kleinste Durchmesser des Innenkonus 40, so daß an der Übergangsstelle eine Stufe 58 gebildet wird. Diese Stufe 58 dient als Anschlag für einen im Führungsabschnitt 56 axial verschieblich gehalterten rohrförmigen Körper 60, der auf seinem äußeren Mantel vorteilhafterweise mit längsverlaufenden Rippen 62 versehen ist, die in gleichen Abständen über dessen Umfang verteilt sind. Der Körper 60 stützt sich über die längsverlaufenden Rippen 62 auf die zylindrische Wand des Führungsabschnitts 56 ab und berührt an seiner konusförmig verjüngten Spitze 64 die Ventilscheibe 46.

In Figur 2 ist nun der Körper 60 in seiner vorgeschobenen Stellung dargestellt, in der der Schlitz 48 der Ventilscheibe 46 durch Spreitzen geöffnet ist. Diese vorgeschobene Stellung wird dadurch erreicht, daß das vorstehend erwähnte, nicht gezeigte konische Anschlußstück eines Schlauchsystems oder einer Spritze den Körper 60 beim Konnektieren nach vorne verschoben hat.

Bei Dekonnektion des Schlauchsystems bzw. der Spritze bewirkt die Rückstellkraft der Ventilscheibe 46 ein Schließen des Schlitzes 48 und damit Rückschieben des Körpers 60.

## Patentansprüche

1. Portkanüle (10) zur materialschonenden Punktion des Septums eines intrakorporal angeordneten Ports, mit einer am distalen Ende (20) angeschliffenen Metallkanüle (12), die an ihrem proximalen Ende (36) in ein Anschlußstück (14) übergeht, dadurch gekennzeichnet, daß in dem Anschlußstück (14) ein den Zugang zu der Metallkanüle (12) keimdicht verschließendes Ventil (46) angeordnet ist, das geometrisch so ausgelegt ist, daß es durch einen in das Anschlußstück hineinragenden Teil eines komplementären Anschlußstückes zur Verbindung mit einer Spritze o.ä. geöffnet werden kann.

2. Portkanüle nach Anspruch 1, dadurch gekennzeichnet, daß das Ventil eine Ventilscheibe (46) mit einem Schlitz (48) ist, die sich quer durch das Anschlußstück (14) erstreckt und in einer ringförmigen Nut (44) gehaltert ist.

3. Portkanüle nach Anspruch 1 oder 2, dadurch gekennzeichnet, daß zwischen der Einführöffnung (54) und der Ventilscheibe (46) ein rohrförmiger Betätigungskörper (60) angeordnet ist, der derart längsverschieblich geführt ist, daß er in seiner zurückgeschobenen Stellung mit seiner der Ventilscheibe (46) zugewandten Vorderseite vor der Scheibe (46) liegt und diese in seiner vorgeschobenen Stellung zur Öffnung des Schlitzes (48) zumindest teilweise durchsetzt.
